# EUROPÄISCHE PATENTSCHRIFT

(11) **EP 2 440 179 B1**
(45) Veröffentlichungstag und Bekanntmachung des Hinweises auf die Patenterteilung: **09.08.2017**
(21) Anmeldenummer: 10744496.0
(22) Anmeldetag: 04.06.2010
(51) Int. Cl.: A61K 9/06, A61K 9/00, A61K 31/19, A61K 31/192, A61K 31/196, A61K 31/197, A61K 31/60, A61K 41/00, A61K 47/12, A61K 47/26, A61K 47/36, A61K 47/32

(54) **ZUSAMMENSETZUNGEN AUF BASIS VON CHITOSAN-OLIGOSACCHARIDEN**
CHITOSAN OLIGOSACCHARIDE-BASED COMPOSITIONS
COMPOSITIONS À BASE D'OLIGOSACCHARIDES DE CHITOSANE

(30) Priorität: 10.06.2009 DE 102009024542
(43) Veröffentlichungstag der Anmeldung: 18.04.2012
(73) Patentinhaber: Teslenko, Alexander, 58095 Hagen (DE); Arivine Pharma Ag, 8008 Zürich (CH)
(72) Erfinder: TESLENKO, Alexander, 58095 Hagen (DE)
(74) Vertreter: Thiel, Christian
(86) Internationale Anmeldenummer: PCT/EP2010/003371
(87) Internationale Veröffentlichungsnummer: WO 2010/142400

(56) Entgegenhaltungen:
- EP-A1- 1 693 051
- WO-A1-03/105797
- FR-A1- 2 702 144
- US-A- 5 730 876
- US-A- 5 830 883

## Beschreibung

Die Erfindung betrifft eine hautpenetrierende (transdermale, dermatologische) Zusammensetzung enthaltend ein Chitosan-Oligosaccharid sowie ein oder mehrere Arzneimittel.

Bei der Verabreichung von Arzneimitteln treten häufig unerwünschte Nebenwirkungen auf. Eine Nebenwirkung ist eine beobachtete oder unbeobachtete Wirkung eines Arzneimittels, die nicht zu seinen beabsichtigten, erwünschten (Haupt-)Wirkungen gehört. Auch Wechselwirkungen mit anderen Substanzen, Folgen von Überdosierungen sowie die Entwicklung von Abhängigkeiten können zu den Nebenwirkungen gezählt werden. Das Spektrum möglicher Nebenwirkungen von Medikamenten reicht von relativ harmlosen Begleiterscheinungen (z. B. Müdigkeit) bis hin zu Wirkungen, deren Schaden den Nutzeffekt des Medikamentes übersteigt.

In Deutschland sterben jedes Jahr mehr als 50.000 Menschen an unerwünschten Arzneimittelnebenwirkungen. 1.100 bis 2.200 Menschen sterben in Deutschland jährlich an gastrointestinalen Komplikationen (Schätzungen). Die Dunkelziffer dürfte deutlich höher liegen. Die gesetzlichen Krankenkassen wenden jährlich fast 125 Mio. Euro für die Behandlung gastrointestinaler Nebenwirkungen der NSAR (nichtsteroidale Antirheumatika) auf.

Gerade bei der Verabreichung von Entzündungshemmern bzw. Analgetika und Antiphlogistika über den Gastrointestinaltrakt kommt es häufig zu Nebenwirkungen. So wird beispielsweise Diclofenac von vielen Patienten bei oraler Verabreichung nur schlecht vertragen.

### BESTÄTIGUNGSKOPIE

Pharmazeutische Stoffe können allein oder in Gegenwart eines Trägers verabreicht werden. Solche Träger können verschiedenen Zwecken dienen, bspw. der kontrollierten Freisetzung von biologisch aktiven Molekülen und dem Targeting biologisch aktiver Moleküle im Hinblick auf spezifische Gewebe.

Aufgabe der vorliegenden Erfindung ist es, Zusammensetzungen zur Verfügung zu stellen, die die Aufnahme von Arzneimitteln über die Haut erlauben.

Diese Aufgabe wird erfindungsgemäß gelöst durch Zusammensetzungen nach Anspruch 1.

Überraschend hat sich herausgestellt, dass es die Kombination eines Arzneimittels mit einem Chitosan-Oligosaccharid ermöglicht, diese Stoffe über die Haut in den menschlichen oder tierischen Körper einzuschleusen, so dass der Gastrointestinaltrakt umgangen werden kann. Gleichzeitig wird vielfach eine Potenzierung der Wirkung des Wirkstoffs beobachtet, d. h. es kann ein Synergismus beobachtet werden.

Ein Synergismus liegt vor, wenn bei der gleichzeitigen Anwendung von zwei oder mehr Wirkstoffen der gemessene Effekt der Kombination größer ist als der der Einzelsubstanzen. Addieren sich die Einzeleffekte, d.h. entspricht die Gesamtwirkung der Summe der Einzelwirkungen, so spricht man von additivem Synergismus. Daneben gibt es einen überadditiven Synergismus (Potenzierung). In diesem Fall ist der Gesamteffekt größer, als es aufgrund der Addition der Einzeleffekte zu erwarten wäre.

Die Erfindung betrifft Haut penetrierende Zusammensetzungen auf Basis von Chitosan-Oligosacchariden und deren Anwendungen in der Medizin, Tiermedizin und Kosmetik. Sie basiert auf den Haut penetrierenden Eigenschaften von Chitosan-Oligosaccharidverbindungen und dadurch vorhandene Möglichkeiten, den Körper mit Wirkstoffen zu versorgen und topische Wirkungen zu erzielen.

Vom strukturellen Aufbau der Haut her sind prinzipiell zwei Möglichkeiten für den dermalen Wirkstofftransport bekannt: Durch das Stratum corneum und über die Hautanhangsgebilde (Follikel, Glandeln). Aufgrund des geringen Oberflächenanteils (etwa 0,1 bis 1,0 %) scheinen der transglanduläre und transfollikuläre Weg von untergeordneter Bedeutung. Neben physikalischen Methoden (Sonophorese, Elektrophorese, Elektroporation u. a.) oder Schaffung von Okklusionsbedingungen bietet sich die Möglichkeit, durch Anwendung von Penetrationsverbesserern (Enhancer) die Barrierefunktion der Haut herabzusetzen. Eine Modulation der Barriereeigenschaften der Hornschicht kann die Penetration und Permeation eines Arzneistoffs erhöhen oder eine Anreicherung in einer definierten Hautschicht bewirken.

Die Erhöhung der Diffusionsgeschwindigkeit kann durch verschiedene Maßnahmen herbeigeführt werden. Die Diffusion wird u. a. durch die Dicke der Membran, den Hydratationszustand der Haut (Okklusion), die Temperatur, den Verteilungskoeffizienten des Wirkstoffs zwischen Grundlage und Hornhaut und der Wirkstoffkonzentration mitbestimmt. Insbesondere die Beeinflussung der Hydratation durch topische Applikation einer Formulierung ist von Bedeutung. Eine Hydratation der Korneozyten nach einer Okklusion öffnet den transzellulären Penetrationsweg. Die Solvatisierung der polaren Kopfgruppen der Hautlipide führt einerseits zur Fluidisierung der Lipiddoppelschichten, andererseits durch Erhöhung der Wasserbindungskapazität zu einer Ausweitung der wässrigen Domänen der Lipiddoppelschicht. Dadurch wird die Penetration sowohl von lipophilen als auch von hydrophilen Stoffen erleichtert.

Die Verwendung von Chitosan als Mittel zur Verabreichung von Arzneimitteln über die Schleimhaut wurde bereits beschrieben (US 2005/0085440 A1, US 2008/02488991 A1, US 2006/0293216 A1). Die Entwicklung von mucoadhäsiven Arzneimittelformulierungen spielte in den letzten 20 Jahren eine große Rolle, da eine verlängerte Kontaktzeit von Arzneistoff und Mucosa die Aufnahme und somit die Bioverfügbarkeit verbessert, insbesondere für schlecht resorbierbare Moleküle. Neu sind jedoch Formulierungen von Chitosan zur dermalen Verabreichung von Arzneimitteln.

Chitosan-Oligosaccharide können durch Depolymerisation von Chitosan mittels Verwendung von Enzymen und Säuren, insbesondere Essigsäure, hergestellt werden. Die weitere Aufbereitung kann mit Hilfe eines Ionenaustauschers erfolgen. Das entstehende Produkt mit freien Aminogruppen, ist in der Lage, mit gewünschten Stoffen verschiedener Klassen Addukte über Ionen-, Chelat- oder andere Molekülbindungen in wässriger Lösung oder in Wasser-LösungsmittelGemischen zu bilden. Dadurch kann eine verbesserte und synergistische Wirksamkeit erreicht werden.

Chitin und Chitosan sind von großer Bedeutung in vielen Bereichen der modernen Medizin. Obwohl Chitin keine Verbindung ist, die im menschlichen Körper vorkommt, spielt es im Vergleich zu anderen natürlichen Polysacchariden eine außergewöhnliche Rolle. In vielen Fällen wirkt sich die Verabreichung von Chitinderivaten beim Menschen positiv aus, so u. a. auf Heilungsprozesse, dient aber auch der Prävention. Die besondere Attraktivität von Chitin/Chitosan in der Medizin liegt darin, dass es sich um ein bioabbaubares, verträgliches und biokompatibles Aminopolysaccharid handelt, das auch eigene biologische Aktivitäten zeigt. Chitosan kann aus Chitin durch Deacetylierung gewonnen werden, wobei die Deacetylierung alkalisch oder enzymatisch erfolgen kann. Eine alkalische Deacetylierung ist beispielsweise mit Natronlauge, eine enzymatische mit Hilfe von Chitin-Deacetylase möglich. Der Grad der Deacetylierung kann dabei variieren; sofern im Gesamtmolekül mehr deacetylierte als acetylierte 2-Amino-2-desoxy-β-D-glucopyranoseeinheiten vorliegen, spricht man von Chitosan, d. h. auch Chitosan enthält häufig noch in nicht unerheblichem Maße acetylierte Aminofunktionen.

Chitosan-Oligosaccharide, auch Chitooligosaccharide oder COS genannt, sind Depolymerisate von Chitosan mit unterschiedlichen Molekulargewichten. Die Herstellung von COS ist bekannt. Chitosan kann leicht durch unterschiedliche Verfahren depolymerisiert werden u.a. durch Radiolyse, durch UltraschallBestrahlung oder durch Hydrolyse mit anorganischen Säuren. Insbesondere durch enzymatischen Abbau kann sowohl Chitin als auch Chitosan depolymerisiert werden. Für die enzymatische Depolymerisation verwendet man vor allem Hydrolase sowie Cellulase, Chitinase, Chitosanase, Lysozym und auch z.B. Papain (Lihn H. et al.: Preparation of chitosan oligomers by immobilized papain., Enzyme Microb. Technol. 2002, 31, 588-92).

Die biologische Aktivität von COS kann insbesondere von ihrem Deacetylierungsgrad, Molekulargewicht und ihrer Molekulargewichtsverteilung abhängen. Der Hydrolysegrad und die molekulare Verteilung der entstehenden Oligosaccharide wird im wesentlichen durch die Art des Hydrolyseverfahrens beeinflusst.

Bei den durch Hydrolyse hergestellten COS sind die Aminogruppen des Glucosamins protoniert und elektrostatisch an ein Anion der bei der Hydrolyse eingesetzten Säure gebunden. Die Chitosan-Hydrolyse in anorganischen Säuren (Salpetersäure, Schwefelsäure oder Salzsäure) zur Herstellung des wasserlöslichen Oligosaccharids führt also zu Oligosaccharidverbindungen mit dem entsprechenden anorganischen Gegenion, wie Nitrat, Sulfat oder Chlorid Die enzymatische Hydrolyse von Chitosan wird in sauren Puffer-Lösungen z. B. mit Papain in Natriumacetat/Essigsäure-Puffer durchgeführt, siehe Lin. H. et al.: Preparation of chitosan oligomers by immobilized papain; Enzyme Microbiol. Technol., 2002, 31, 588-592. Dadurch bildet sich COS-Acetat. Allerdings ist hier nur eine begrenzte Steuerung hinsichtlich des Hydrolysegrades und damit des Molekulargewichts möglich.

Dennoch wurde das auf bisher beschriebene Weise erhaltene COS pharmakologisch untersucht. So wurde in zahlreichen Untersuchungen nachgewiesen, dass nicht Chitin/Chitosan, sondern deren Spaltprodukte, nämlich Chitosan-Oligosaccharide (COS), biologische Aktivität zeigen, vgl. Muzzarelli R.A.A.(Ed.): Chitosan per os, from dietary supplement to drug carrier. Atec Edizioni, 2000. Nach neuestem Kenntnisstand spielt im Rahmen der menschlichen Physiologie die enzymatische Aktivität von hydrolytischen Enzymen, wie Chitinase, Lysozym oder Hexoaminidase, eine entscheidende Rolle. Auch Makrophagen sind in der Lage, eine große Menge von diesen Enzymen im menschlichen Körper zu produzieren, siehe Muzzarelli R.A.A.: Human enzymatic activities related to the therapeutic administration of chitin derivates, CMLS. Cellular Molec. Life Sci. 1997, 53, 131-140.

U. a. konnten folgende biologische Aktivitäten von COS nachgewiesen werden:
COS zeigen keine cytotoxische Wirkung und werden wegen ihres niedrigen Molekulargewichtes schnell im Magen resorbiert. Sie gelangen im Gegensatz zu Chitosan innerhalb kurzer Zeit in die Blutbahn und in verschiedene Organe des Körpers.

Sie aktivieren Immunzellen, u.a. Makrophagen, durch Anwesenheit von Rezeptoren für die COS an ihrer Oberfläche. Ferner üben sie eine antioxidative Aktivität aus, insbesondere durch Fangen von ROS (Reactive Oxygen Species).

COS sind auch in der Lage, Membranproteine von Zellen zu modifizieren und zelluläre Prozesse zu beeinflussen.

COS dienen im Körper als Depot von GlcNH₂ und GlcNAc, notwendig für die Biosynthese von Glykosaminoglycanen und Glycoproteinen. Sie weisen auch antibakterielle Wirkungen auf.

Zusammensetzungen, die Chitosan-Oligosaccharide zusammen mit Nicotinsäure, Acetylsalicylsäure, Milchsäure, Glutaminsäure, Nifluminsäure, Bernsteinsäure, Ascorbinsäure, Acexamsäure, Glycin, L-Carnitin, γ-Amino-β-phenylbernsteinsäure, Adenosinphosphat, Cocarboxylase, Aspartam, Gemfibrozil, Aminocapronsäure, Vitamin B₃, Methionin, einem Salz der vorgenannten Verbindungen, Antibiotika oder Polysaccharide enthalten, wurden bereits in der EP 1 659 182 A1 beschrieben, die Kombination mit COS soll hier jedoch nur der Erhöhung der Stabilität der Wirkstoffe dienen, die Ermöglichung der transdermalen Verabreichung spielt keine Rolle.

Auch die Komposition von COS mit Acetylsalicylsäure, Insulin und Antibiotika wurde beschrieben (US 2006/0293216 A1). Allerdings werden die beschriebenen Addukte nur mucosal (peroral und intranasal) angewendet. Ebenso wurde ein Buprenoprin enhaltender Chitosan-Polaxomer- bzw. Chitosan-Hydropropylmethylcellulose-Komplex für die intranasale Anwendung offenbart (US 2005/0085440 A1), ebenso ein physiologisches Chitosan-Polyphosphat-Gel für mucosale und transmucosale Verabreichung mit Hormonen und Antimigräne-Arzneistoffen (US 2008/0248991 A1). WO 03105797 offenbart zur Haar- und Hautbehandlung geeignete Mikro-Emulsionen enthaltend ein Arzneimittel und ein COS. Auch die COS-Addukte mit einigen Antibiotika und Antiphlogistika in Form von Tabletten oder Implantaten wurden beschrieben (US 5,900,408, WO 2006/087028 A1). Neu ist jedoch die transdermale Anwendung von hautpenetrierenden Arzneimittel-Chitooligosaccharid-Addukten.

Die Herstellung der erfindungsgemäßen Zusammensetzung erfolgt, indem Chitosan geeigneter Herkunft mittels eines Enzyms und einer Säure, vorzugsweise Essigsäure, hydrolysiert und das erhaltene Produkt einem Ionenaustausch unterzogen wird, so dass der Acetatgehalt auf 2 bis 5 Gew.-% reduziert wird. Auf diese Weise werden Verbindungen erhalten mit einem mittleren Molekulargewicht von 240 bis 200.000 Da. Bevorzugt liegt das mittlere Molekulargewicht zwischen 240 und 50.000 Da. Durch Zugabe erwünschter pharmazeutischer Wirkstoffe bilden sich mit gewünschtem Substitutionsgrad ionische und nicht ionische Chitooligosaccharidverbindungen. Anschließend erfolgt in der Regel eine Sprüh- oder Gefriertrocknung. Anstelle der Verwendung eines Ionenaustauschers zur Reduzierung des Gehalts an Acetationen ist auch das wiederholte Sprühtrocknen und Lösen in Wasser möglich, wie dies in der EP 1 659 182 A1 beschrieben wird.

Es hat sich überraschend gezeigt, dass die erfindungsgemäßen Chitosan-Oligosaccharid-Zusammensetzungen sehr schnell und tief in die Haut durch das Stratum corneum eindringen und die Haut mit den o.g. Stoffen anreichern. Auf diese Weise kann man den Körper mit pharmazeutischen oder kosmetischen Wirkstoffen behandeln. Es hat sich ebenso gezeigt, dass die Chitosan-Oligosaccharid-Zusammensetzungen mit pharmazeutischen und kosmetischen Wirkstoffen synergistische Wirkungen zeigen, so dass die Gesamtwirkung potenziert wird. Dadurch besteht die Möglichkeit, die therapeutische Dosis herabzusetzen und unerwünschte Nebenwirkungen zu reduzieren.

Die erfindungsgemäßen Zusammensetzungen liegen als Mikroemulsion von. Feste Zubereitungen oder Zubereitungen auf Partikelbasis sind hingegen ungeeignet, da eine transdermale Aufnahme auf diese Weise nicht erfolgen kann. Die Zubereitungen können Hilfsstoffe enthalten, wie sie üblicherweise in solchen Zubereitungen verwendet werden, z. B. Konservierungsmittel, Bakterizide, Antioxidantien, Tenside, Farbstoffe, Schaumverhinderer, Verdickungsmittel, Emulgatoren, Fette, Öle usw. Auch die Darreichung der erfindungsgemäßen Zusammensetzung in verkapselter Form ist möglich, z. B. liposomal verkapselt.

Bei der an sich bekannten Hydrolyse von Chitosan mittels eines Enzyms und einer Säure, vorzugsweise Essigsäure wird normalerweise eine breite Molekulargewichtsverteilung der erhaltenen Mischung erhalten (vgl. Tab. 1). Erfindungsgemäß wird nunmehr diese Produktmischung neutralisiert mit Hilfe eines Ionenaustauscherharzes und aufkonzentriert. Für die Gewinnung von Fraktionen mit gewünschter Molekulargewichtsverteilung kann eine Fraktionierung mit Hilfe von Ultrafiltrationsverfahren eingesetzt werden.

Im Laufe dieses Prozesses verlieren die Chitosan-Oligosaccharide einen gewissen Anteil (60 bis 98 %) der Essigsäure. Auf diese Weise ist es möglich, eine derartige Reduktion des Acetatgehaltes zu erreichen, dass bei Umsetzung mit anderen ionischen oder nicht ionischen Substanzen die jeweils gewünschten Addukte erhalten werden können, obwohl normalerweise die mit dem Anion der Hydrolysesäure erhaltenen Komplexe vorliegen.

Ebenso sind erfindungsgemäß nicht ionische COS-Addukt-Verbindungen möglich, die durch Chelat-Bildung oder durch andere Wechselwirkungen mit den wie beschrieben hergestellten wasserlöslichen COS gebildet werden können.

Die Umsetzung zu den Addukten erfolgt normalerweise in wässriger Lösung mit anschließender Trocknung (Sprühtrocknung, Lyophilisierung).

Als Ausgangsmaterial im beschriebenen Verfahren wird als Quelle für die Herstellung von COS Chitosan von Krebstieren (Krabben, Garnelen, Krill u.a.), Insekten (Bienen, Seidenwürmer u.a.), aus Basidomyzeten, aus mikroskopischen Pilzen und Mikroalgen verwendet. Das daraus erhaltene Chitosan ist bekannt.

Insbesondere geeignet ist als Ausgangsmaterial Chitosan mit einem Molekulargewicht von 10 kDa bis 1000 kDa, bevorzugt von 10 kDa bis 500 kDa und insbesondere von 100 kDa bis 300 kDa, das einen Deacetylierungsgrad von ca. 85 Gew.-% aufweist. Es können unterschiedliche Enzyme eingesetzt werden. Hierzu gehören insbesondere hydrolytische Enzyme wie Cellulase, Chitinase, Chitosanase oder Papain, bevorzugt Chitinase, Chitosanase oder Papain. Zu den eingesetzten Säuren zählen vor allem Essigsäure und Propionsäure. Besonders bevorzugt ist Essigsäure.

Ganz besonders bevorzugt wird die Hydrolyse von Chitosan (enzymatische Depolymerisation) vorgenommen mittels Chitosanase und Essigsäure. Die Bedingungen hierfür sind an sich bekannt.

Erfindungsgemäß besonders geeignet ist die Kombination eines Chitosan-Oligosaccharides mit einem entzündungshemmenden Wirkstoff, einem Analgetikum, einem Cytostatikum, einem Photosensibilisator, einem Hautarzneimittel, einem Antiphlogistikum und/oder einem Antirheumatikum. Die Zusammensetzung dient besonders der therapeutischen oder prophylaktischen Behandlung von Krankheiten, die einen entzündlichen Hintergrund haben. Auch für die Wundheilung sind die erfindungsgemäßen Zusammensetzungen von Vorteil.

Die Erhaltung der physiologischen Homöostase des Körpers unterliegt einer Vielzahl von Regelkreisen. Auf schädliche chemische oder physikalische Noxen, Infektionen oder Verletzungen reagiert das Gefäßbindegewebe am Ort der Schädigung mit einer Entzündung. Der biologische Zweck der entzündlichen Reaktion ist die Abwehr aller Noxen, die die Intaktheit und Funktionsfähigkeit des Organismus bedrohen, sowie die Wiederherstellung der geschädigten Struktur. Die dabei ablaufenden Vorgänge sind durch eine enge Vernetzung von vaskulären und zellulären Reaktionen sowie Antigen-spezifischen und Antigenunspezifischen Abwehrreaktionen gekennzeichnet. Als sog. Kardinalsymptome der (akuten) entzündlichen Reaktionen treten Wärme, Rötung, Schwellung, Schmerz und gestörte Funktionen auf. Diese Symptome beruhen auf der Freisetzung von Entzündungsmediatoren: Histamin, Bardykinin, Prostaglandinen, Leukotrienen, Komplementfaktoren, reaktiven Sauerstoffspezies (ROS) u. a.

Schon zu Beginn der Entzündung kann diese durch Beseitigung der Noxen wieder abklingen. Neben der Emigration von Blutzellen in den extrazellulären Raum dienen eine Proliferation von Histiozyten und Fibroblasten primär der Bekämpfung der Schädigung sowie der Wiederherstellung des ursprünglichen Zustandes. Diese können sich jedoch auch negativ auswirken (chronische rheumatische Entzündungen). Je nach Verlauf einer Entzündung lassen sich perakute, akute, chronische und rezidivierende Entzündungen unterscheiden.

Die Entzündungsreaktion ist ein sehr komplexes Wirkungsnetz unter Beteiligung verschiedener humoraler Faktoren, Zellen, reaktionsfördernder und reaktionshemmender Mediatoren sowie der Zielorgane der verschiedenen Reaktionsketten. Die medikamentöse Behandlung der entzündlichen Erkrankungen basiert auf der Beeinflussung solcher Reaktionsketten.

Die Prostaglandine sind an der Entstehung der Schmerzen, des Fiebers und den entzündlichen Reaktionen wesentlich beteiligt. Es ist somit nachvollziehbar, dass Verbindungen, die die Bildung von Prostaglandinen blockieren, gleichzeitig schmerzunterdrückend, fiebersenkend und entzündungshemmend wirken. Die am häufigsten angewendeten schmerzlindernden und antientzündlichen Medikamente gehören zur Gruppe der NSAID.

Bei der topischen Anwendung von nicht-steroidalen Antirheumatika, z. B. in Form von Salben, Gelen oder Sprays, können die bei systemischer Gabe auftretenden Nebenwirkungen reduziert werden. Obwohl eine Resorption in tiefere Gewebeschichten erfolgt und eine gewisse Anreicherung in entzündlichen Gebieten gezeigt werden konnte, verbleibt die Hauptmenge der NSAID-Präparate auf der Hautoberfläche. Die Plasmaspiegel liegen weit unter denen, die bei gleicher Dosierung nach oraler Gabe gemessen werden. Trotz ihrer sehr häufigen Anwendung wird die therapeutische Wirksamkeit dieser Präparate unterschiedlich beurteilt.

Durch die Applikation einer Zusammensetzung, die ein Arzneimittel, insbesondere ein antientzündliches Mittel, Antiphlogistikum oder Analgetikum enthält, lassen sich jedoch deutlich höhere Plasmaspiegel erzielen.

Als antientzündliche Mittel können Salicylate, Indole, Propionate, Pyrazole, Fenamate und Anthraniline zum Einsatz kommen.

Das Antiphlogistikum oder Antirheumatikum kann beispielsweise eine Verbindung aus der Gruppe der Glucocorticoide, Pyrazolidine, Essigsäurederivate, Oxicame, Coxibe, Salicylate, Immunsuppressiva/Immunmodulatoren, Fenamate oder Propionsäurederivate sein. Als Pyrazolidine kommen z. B. Clofezon, Kebuzon, Mofebutazon, Oxyphenylbutazon, Phenylbutazon und Pyrazinobutazon, als Essigsäurederivate z. B. Aceclofenac, Acemetacin, Alclofenac, Bufexamac, Bumadizon, Carbamoylphenoxyessigsäure, Diclofenac, Difenpiramid, Etodolac, Fentiazac, Indometacin, Ketorolac, Lonazolac, Oxametacin, Proglumetacin, Sulindac, Tolmetin oder Zomepirac in Frage.

Oxicame können z. B. sein: Droxicam, Lornoxicam, Meloxicam, Piroxicam oder Tenoxicam.

Mögliche Fenamate sind: Etofenamat, Flufenaminsäure, Meclofenaminsäure, Mefenaminsäure, Tolfenaminsäure.

Als Propionsäurederivate kommen z. B. Alminoprofen, Benaxaprofen, Carprofen, Dexibuprofen, Dexketoprofen, Fenbufen, Fenoprofen, Flunaxaprofen, Flurbiprofen, Ibuprofen, Ibuproxam, Indoprofen, Ketoprofen, Naproxen, Oxaprozin, Pirprofen, Surprofen und Tiaprofensäure in Frage.

Das Analgetikum kann eine Verbindung aus der Gruppe der Opioide, Phenylpiperidinderivate, Diphenylpropylaminderivate, Benzomorphanderivate, Oripavinderivate und Morphinanderivate sein.

Als Alkaloide kommen z. B. Morphin, Opium, Hydromorphon, Nicomorphin, Oxycodon, Dihydrocodein, Diamorphin und Papaveretum in Frage.

Phenylpiperidinderivate können sein: Ketobemidon, Pethidin, Fentanyl.

Diphenylpropylaminderivate sind z. B.: Dextromoramid, Piritramid, Dextropropoxyphen, Bezitramid, Methadon.

Benzomorphanderivate sind beispielsweise Pentazocin und Phenazocin.

Ein typisches Oripavinderivat ist Buprenorphin. Morphinanderivate sind z. B. Butorphanol und Nalbuphin. Weitere Opioide können sein: Tilidin, Tramadol, Dezocin und Meptazinol.

Weitere verwendbare Analgetika oder Antipyretika sind Rimazolium, Glafenin, Floctafenin, Viminol, Nefopam, Flupirtin und Ziconotid.

Zu berücksichtigen ist, dass eine scharfe Abgrenzung zwischen Antiphlogistika und Analgetika häufig nicht möglich ist. Darüber hinaus können Analgetika/Antiphlogistika häufig auch beispielsweise als Antirheumatika oder Antiallergika eingesetzt werden.

Zu den Analgetika/Antiphlogistika, die als Bestandteil der erfindungsgemäßen Zusammensetzung in Frage kommen, zählen insbesondere:
Morphin, Codein, Dihydrocodein, Oxycodon, Fentanyl, Piritramid, Pethidin, Pentazocin, Methadon, Buprenorphin, Tramadol, Flupirtin, Nalbuphin, Nefopam, Metamizol, Paracetamol, Phenylbutazon, Mofebutazon, Acemetacin, Ibuprofen, Naproxen, Diclofenac, Ketoprofen, Ketorolac, Clofezol, Lonazolac, Aceclofenac, Indometacin, Sulindac, Tolmetin, Oxaprozin, Dexibuprofen, Mefenamin, Proglumetacin, Tiaprofensäure, Surprofen, Dexketoprofen, Lornoxicam, Meloxicam, Piroxicam, Penicillinamin, Chloroquin, Cromoglicinsäure, Nedocromil, Methotrexat, Auranofin, Natriumaurothiomalat, Oxaceprol, Leflunomid, Sulfasalazin, Valdecoxib, Celecoxib, Lumeracoxib, Ademetionin, Hyaluronsäure, Rofecoxib, Epibatidin, Boswellsäure, Methylsalicylsäure, Diflunisal, Phenazon, Proxyphenazon, Aescin, Escin, Heparin, Benzydamin, Bufexamac, Flurbiprofen, Oxyphenbutazon, Salicylamid, Tolfenaminsäure, Clobetasol, Triamcinolonacetonid, Triamcinolonhexacetonid, Betamethason, Dexamethason, Prednisolon, Hydrocortison, Fluticason, Liponsäure, Budesonid, Montelukast, Gentisinsäure, Salicylsäure, 5-Aminosalicylsäure, Olsalizin, Anthranilsäure, Flufenaminsäure sowie deren Säuren, Ester und Derivate.

Auch viele pflanzliche Extrakte und daraus gewonnene Phytopharmaka zeigen eine entzündungshemmende oder schmerzlindernde Wirkung. Beispiele für Phytopharmaka, die erfindungsgemäß mit Chitosan-Oligosacchariden kombiniert werden können, sind:
Glykoside: Saponine, steroidale Glykoside, Triterpen-Glykoside (Glyzyrrhitinsäure), Thioglykoside (Allicin, Allinin, Sinigrin), Anthraglykoside (Salidrosid, Eludin)
Alkaloide: Koffein, Berberin, Capsaicin, Ecgoin, Morphin, Amirin, Xantin, Salsolin, Stahydrin, Theobromin
Phenolverbindungen: Cumarine, Xantane, Chinone (Benzochinon, Antrachinon, Ubichinon-Q10), Lignan
Polyphenole: Phenolsäuren, Ferulasäure, Ellagsäure, Hydroxyzimtsäure
Flavonoide (Flavonole, Flavonone und Flavone): Quercetin, Katechin, Gesprin, Amygdalin, Arbutin, Genestin,
Anthocyanine: Malvidin, Peonin,
Gerbstoffe und Tannine: Tannin, Procyanidin
Phytoöstrogene: ß-Systosterol, Stigmasterol, Cimicifugosid

Die Extrakte und Phytopharmaka können aus einer Vielzahl von Pflanzen gewonnen werden, beispielsweise Weihrauch, Ingwer, Arnika, Birke, Weide, Curcuma, Hagebutte, Beinwell, Nachtkerze, Kamille, grüner Tee, Lakritze, Lavendel, Rosmarin, Aloe Vera, Teufelskralle, Pfeffer, Efeu, Eibisch oder Eukalyptus.

Obgleich als mit den COS zu kombinierende Arzneimitteln entzündungshemmende Mittel, Analgetika und Antiphlogistika bevorzugt sind, kann es sich bei dem Arzneimittel auch um ein Antifibrinolytikum, ein Cytostatikum, einen Photosensibilisator, ein Hautarzneimittel, ein Hormon, eine Aminosäure, ein Peptid, ein Vitamin oder ein Antioxidanz handeln.

Antifibrinolytika sind z. B. ε-Capronsäure, Tranexamsäure, para-Aminomethylbenzolsäure oder Lysin.

Hormone können z. B. sein: Cortison, Testosteron, Östradiol, Melatonin, Dehydroepiandrosteron und entsprechende Derivate.

Aminosäuren bzw. Aminosäurederivate können sein: herkömmliche Aminosäuren wie Cystein und Arginin, aber auch nicht proteinogene Aminosäuren wie Creatin, Glutathion, Hydroxyprolin, Taurin, Citrullin, Ornithin, γ-Aminobuttersäure, Thyroxin, Homocystein, Aszaserin, Adenosylmethionin.

Bei den Peptiden kann es sich um Di-, Tri-, Tetra- und Polypeptide handeln, z. B. Karnison, Glutathion, Ephitalamin u. a.

Vitamine bzw. vitaminähnliche Stoffe können sein: Thiamin, Riboflavin, Pantothensäure, Pyrridoxin, Folsäure, Kobalamin, Orotsäure, para-Aminobenzoesäure, Inosit, Bioflavonoide, Biotin, Alfa-Tocopherol, Retinol und Derivate, Beta-Karotin, Liponsäure.

Bei der Kombination mit Cytostatika macht sich positiv bemerkbar, dass Chitosan von Krebszellen stärker absorbiert wird als von normalen Körperzellen. Das Medikament kann daher gezielt zu den Krebszellen gebracht werden. Dies ist auch bei der Kombination mit Photosensibilisatoren für die Photodynamische Therapie (PDT) von Bedeutung. Hierbei wird dem Patienten ein Photosensibilisator verabreicht, der sich selektiv im Tumor anreichert. Anschließend wird der Tumor mit Licht geeigneter Wellenlänge bestrahlt. Dabei werden durch photophysikalische Prozesse Substanzen (bspw. Radikale, Singulett-Sauerstoff) erzeugt, die aufgrund der Tumorselektivität des Photosensibilisators gezielt den Tumor schädigen.

Über die Kombination mit Hautarzneimitteln ist die Behandlung von Hauterkrankungen, bspw. Neurodermitis oder Psoriasis möglich. Die COS können beispielsweise mit Corticosteroiden kombiniert werden.

Es können auch mehrere einzelne oben beschriebene Substanzen mit den COS kombiniert werden.

Die Adduktsubstanzen (Wirkstoffe) werden erfindungsgemäß mit den COS, die freie Aminogruppen enthalten, verknüpft, indem man diese in Wasser, z. B. in einer Konzentration von 1 bis 30 Gew.-%, insbesondere 5 bis 25 Gew.-%, und ganz besonders 8 bis 15 Gew.-%, z. B. 10 Gew.-% löst und sodann die zu addierende Substanz unter Rühren bei Temperaturen von 20 bis 60°C in einer jeweils geeigneten Menge mit oder ohne Lösungsmittel hinzufügt und das erhaltene Produkt sprüh- oder gefriertrocknet.

Es ist weiterhin von Vorteil, wenn der zugesetzte Wirkstoff eine Säure ist, da in diesem Fall eine ionische Verbindung mit dem Chitosan-Oligosaccharid in Form eines Salzes erhalten wird. Mit einer nicht-ionischen Verbindung kann sich auch ein Komplex ausbilden. Möglich sind auch Komplexe mit Chelatverbindungen, wobei das Vorhandensein von Metallionen hilfreich sein kann. Diese können beispielsweise in einer Konzentration zwischen 0,01 und 5 Gew.-% vorliegen.

In der erfindungsgemäßen Zusammensetzung beträgt der Anteil des Arzneimittels 0,01 bis 40 Gew.-%, bevorzugt 3 bis 35 Gew.-% und besonders bevorzugt 7 bis 30 Gew.-% bezogen auf das Chitosan-Oligosaccharid. Der Anteil der Essigsäure bezogen auf das Chitosan-Oligosaccharid beträgt 2 bis 5 Gew.-%.

### Beispielhafte Erläuterung des Herstellungsverfahrens:

Durch enzymatische Hydrolyse mit Chitosanase/Essigsäure (pH-Wert: 4,5; Dauer: 5 Stunden; 50 °C) wird Chitosan zu COS-Acetat mit einer breiten Molekulargewichtsverteilung, wie in Tabelle 1 dargestellt, depolymerisiert. Es ist dabei möglich, Chitosan mit einem Deacetylierungsgrad von 60 bis 95 % zu verwenden. Die Analyse erfolgt chromatographisch:
HPLC-System "Bio-Rad" mit einem refraktometrischen Detektor, Säule: Bio-Rad 30XL (7,5x300 mm), Eluent: Phosphatpuffer (0,05 M, pH-Wert: 3,0 mit 0,05 M NaNO₃). Fließgeschwindigkeit: 1,1 ml/min. Für die Kalibrierung wurden Dextrane von 20.000 bis 250.000 Dalton (Pharmacia Fine Chemicals) und Maltooligosaccharide der Fa. Sigma verwendet.

**Tabelle 1: Molekulargewichtsverteilung von COS**

| Molekulargewicht/Da | Anteil/Gew.-% |
|---|---|
| mehr als 315.000 | keine |
| von 125.000 bis 315.000 | 0,3 |
| von 45.000 bis 125.000 | 1,2 |
| von 15.000 bis 45.000 | 2,6 |
| von 5.500 bis 15.000 | 6,6 |
| von 2.000 bis 5.500 | 11,1 |
| von 900 bis 2.000 | 15,1 |
| von 420 bis 900 | 24.2 |
| von 240 bis 420 (Dimere) | 23.8 |
| 240 (Monomer) | 15,1 |

Wie hieraus ersichtlich, resultiert zunächst eine breite Verteilung.

Durch Anwendung eines Ionenaustauschers können andere Chitosan-Oligosaccharid-Verbindungen als Acetate hergestellt werden. Im Laufe dieses Prozesses verliert COS-Acetat einen gewissen Anteil an Essigsäure. Abhängig von der Prozessdauer und COS-Konzentration in der Lösung verliert es 60 bis 98 % der gebundenen Essigsäure. Die entstandenen wasserlöslichen COS weisen 60 bis 98 % freie Aminogruppen der Glucosaminoglucose-Einheiten auf.

Ein auf diese Weise erhaltenes wasserlösliches COS-Produkt mit einem Anteil von mindestens 60 % an freien Aminogruppen kann wie beschrieben mit gewünschten Stoffen aus verschiedenen chemischen Klassen eine Ionenbindung, eine Chelat-Bindung oder durch andere Wechselwirkungen hervorgerufene Bindungen in wässriger Lösung eingehen. Der Nachweis der COS-Verbindungen wurde durch verschiedene analytische Methoden geführt.

Der Nachweis einer ionischen Bindung wurde durch potentiometrische Titrierung, Nuclear Magnetic Resonance (NMR) und chemische Elementaranalyse von COS-Verbindungen durchgeführt. Die Titrationskurven der dialysierten Lösungen zeigen die Anwesenheit von Essigsäure (von 2 bis 8 Gew.-% abhängig vom Herstellungsverfahren) und gebundenen Anionen (von 3 bis 40 Gew.-% der verwendeten Säuren oder Stoffe mit aktivem Wasserstoff).

### Anwendbarkeit

Die erfindungsgemäßen Zusammensetzungen dienen dem Einsatz in der Medizin, insbesondere zur Schmerzlinderung bzw. Entzündungshemmung im menschlichen oder tierischen Körper. Die Anwendung erfolgt über die Haut, d. h. die Haut penetrierenden Eigenschaften der Chitosan-Oligosaccharide werden ausgenutzt, wobei die COS neben ihren eigenen positiven Wirkungen gewissermaßen als Träger zum Transport der Wirkstoffe dienen. Zusätzliche synergistische Wirkungen erlauben durch Herabsetzung der Wirkdosis von Medikamenten (Wirkstoffen) die Minimierung oder Vermeidung unerwünschter Nebenwirkungen.

Zur Applikation werden geeignete galenische Formen gewählt wie Gels, Salben, Cremes, Sprays oder Schaum. Die Verabreichung erfolgt abgestimmt auf die Schwere der Erkrankung oder die Art der Prophylaxe unter Beachtung des Gewichts, Alters, Geschlechts und allgemeinen Zustands des Patienten. Die Zubereitungen können Hilfsstoffe enthalten, wie sie üblicherweise in solchen Zubereitungen verwendet werden, z. B. Konservierungsmittel, Bakterizide, Antioxidantien, Tenside, Farbstoffe, Schaumverhinderer, Verdickungsmittel, Emulgatoren, Fette, Öle usw.

### Beispiele

Von den Beispielen sind die Beispiele 7 und 8 erfindungsgemäß und beschreiben COS-haltige Mikroemulsionen.

### Herstellunasprozess

### Beispiel 1

In 10 I Wasser werden 150 ml Essigsäure gelöst. Dazu werden 500 g Chitosan mit einem Deacetylierungsgrad von 85 % gegeben. Die Lösung wird bis zur vollständigen Lösung des Chitosans gerührt. Danach wird die Lösung auf 50°C erwärmt und 2,5 g Chitosanase hinzugegeben. Die enzymatische Depolymerisation von Chitosan wird im Laufe von fünf Stunden durchgeführt, so dass sich ein heterogenes Chitooligosaccharid-Acetat (n = 2 bis 20 Glucosamineinheiten, siehe Tabelle 1) bildet. Die resultierende Lösung wird filtriert und danach durch eine Ionenaustauschersäule gepumpt. In dem entstehenden Produkt wird der Anteil vorhandener Essigsäure auf 6,0 Gew.-% reduziert. Hinsichtlich der durch Essigsäure gebundenen Aminogruppen der Chitooligosaccharide entspricht dies 17,0 %.

500 g so erhaltenes Chitooligosaccharid werden in 10 l Wasser gelöst und 180 g Salicylsäure hinzugegeben. Die Lösung wird bis zur vollständigen Auflösung der Salicylsäure gerührt. Das entspricht einer Ionenbindung mit 75 % der freien Aminogruppen der Oligosaccharide. Die entstehende Lösung wird sprühgetrocknet. Das erhaltene Produkt enthält 77 Gew.-% Chitooligosaccharid, 20,5 Gew.-% Salicylsäure und 2,55 Gew.-% Essigsäure.

### Beispiel 2

Die wie in Beispiel 1 beschrieben erhaltenen 1,0 kg Chitooligosaccharid werden in 10,0 l Wasser gelöst und 65,2 g Diclofenac hinzugegeben. Dies entspricht einer Ionenbindung mit 5,0 % der freien Aminogruppen der Oligosaccharide. Die entstehende Lösung wird wie oben beschrieben sprühgetrocknet. Das Produkt enthält 92,6 Gew.-% Oligosaccharid, 4,9 Gew.-% Diclofenac und 2,39 Gew.-% Essigsäure.

### Beispiel 3

Die wie in Beispiel 1 beschrieben erhaltenen 1,0 kg Chitooligosaccharid werden in 10,0 I Wasser gelöst und 190,0 g Ibuprofen hinzugegeben. Dies entspricht einer Ionenbindung mit 20 % der freien Aminogruppen der Oligosaccharide. Die entstehende Lösung wird wie oben beschrieben sprühgetrocknet. Das Produkt enthält 80,2 % Oligosaccharid, 16,3 % Ibuprofen und 3,35 % Essigsäure.

### Beispiel 4

Die wie in Beispiel 1 beschrieben erhaltenen 1,0 kg Chitooligosaccharid werden in 10,0 l Wasser gelöst und 185 g Boswellsäure hinzugegeben. Dies entspricht einer Ionenbindung mit 20 % der freien Aminogruppen der Oligosaccharide. Die entstehende Lösung wird wie oben beschrieben sprühgetrocknet. Das erhaltene Produkt enthält 78,72 % Oligosaccharid, 15,9 % Boswellsäure und 3,15 % Essigsäure.

### Anwendungsbeispiele

### Beispiel 5. Nachweis der Eindringtiefe von COS-Salicylat in die Haut

Schweineohren wurden ohne Hautverletzungen sofort nach der Schlachtung aus einem Schlachthof abgeholt. Die Ohren wurden gründlich mit milder Seife abgewaschen. Unmittelbar danach wurde die subkutane Fettschicht mit dem Skalpell abgetrennt. Die Haut wurde in einem Container bei -20°C für weitere Untersuchungen aufbewahrt. Die Trennung der Epidermis von der Dermis erfolgte vorsichtig nach Erwärmung bei 60°C (1 Minute).

Die Eindringtiefe von COS-Salicylat wurde im Rahmen eines Schälverfahrens an einer Modellhaut bestimmt. Die 10%-ige COS-Salicylat-Lösung wurde auf die Hautoberfläche der Ohren aufgetragen und etwa 1 bis 2 Minuten in die Haut einmassiert. Währenddessen wurden die Ohren bei 35 °C temperiert.

Für die Schälung wurden 15 Tesafilmstreifen (Typ Tesa^{®} Office, Beiersdorf, DE) auf eine Größe von 1,5 cm * 1,5 cm geschnitten und ausgewogen. Die auf dem Schweineohr ausgewählten Untersuchungsfelder von 2 cm * 2 cm wurden nacheinander mit den vorbereiteten Tesafilm-Streifen beklebt und danach abgezogen.

Die Streifen wurden mit Acetonitril bei 60°C im Laufe von 60 Minuten extrahiert. Die Lösungen wurden durch 0,5 µm-Membranen filtriert und mit Hilfe von HPLC analysiert.

Das Shimadzu LC-20A HPLC-System bestand aus einer Spherisorb S5 ODS2-Säule (Waters Co) und UV-Detektor. Mobile Phase war Wasser-Methanol-Acetonitril-Essigsaure.

**Tabelle 2: Mittelwerte der Wiederfindung aus 4 behandelten Schweinehäuten des COS-Salicylat (aus Beispiel 1) und Salicylsäure in einzelnen Hautschichten nach 2 Stunden Applikation.**

| Verteilung in | COS-Salicylat, µg/cm² | Salicylsäure, µg/cm² |
|---|---|---|
| Hornschicht | 45,2 ± 4,8 | 5,3 ± 0,4 |
| Epidermis | 20,4 ± 1,9 | 2,1 ± 0,2 |
| Dermis | 11,3 ± 1,1 | 1,0 ± 0,15 |

Der Gehalt von COS-Salicylat in Epidermis und Dermis wurde mit Hilfe einer Franz-Zelle bestimmt.

Wie ersichtlich erfolgt mit dem erfindungsgemäßen COS-Salicylat ein erheblich besseres Eindringen in tiefere Hautschichten, so dass Wirkstoffe besser und effektiver wirken können.

### Beispiel 6. Schmerzlinderndes COS-Ibuprofen haltiges Gel

Die Wirkung des schmerzlindernden 5%-igen COS-Ibuprofen haltigen Gels (2%-iges Carbopolgel) wurde an 14 freiwilligen Probanden (6 männliche und 8 weibliche Personen im Alter zwischen 23 und 45 Jahren) getestet. Das COS-Ibuprofen-Gel wurde im Vergleich zu der EMLA®-Creme (AstraZeneca) untersucht. Es wurden etwa 2 g EMLA®-Creme und 1,0 ml COS-Ibuprofengel auf 10 cm² Haut aufgetragen. Nach unterschiedlichen Einwirkungszeiten wurde das Schmerzempfinden durch kleine Nadelstiche überprüft. Die beteiligten Personen haben ihre Schmerzempfindung auf einer Schmerzgradskala eingeschätzt (0 - kein Schmerz , 5 - normaler Schmerz und 10 - schwerwiegender Schmerz).

**Tabelle 3: Zeit - Analgetisches Potential der COS-Ibuprofen Gel und EMLA®-Creme.**

| Vehikel | Zeit/min | Schmerzgrad |
|---|---|---|
| COS-Ibuprofen Gel | 15 | 10,0 |
| | 30 | 7,5 |
| | 45 | 3,0 |
| | 60 | 2,0 |
| EMLA®-Creme | 15 | 9,5 |
| | 30 | 9,0 |
| | 45 | 6,0 |
| | 60 | 3,0 |

Das COS-Ibuprofen Gel zeigt eine moderate Analgesie vor Ablauf von 45 Minuten und eine effektive Analgesie nach 45 Minuten, während EMLA®-Creme erst nach 1 Stunde wirkt.

### Beispiel 7. Antirheumatische Wirkung

Die Behandlung von Patienten mit Erkrankungen mit entzündlichem Hintergrund wurde in den Wintermonaten 2008 in einer Rheumapraxis durchgeführt.

Alle Behandlungen wurden unter Anwendung von Mikroemulsionen mit COS-Diclofenac und COS-Boswellsäure (1 zu 1) durchgeführt. Die Mikroemulsion wurde in einer Menge von 0,3 bis 0,5 ml auf die betroffene Stelle mit dem Sprüher (Sauerstoffgenerator, Fa. Meddrop Technology AG) aufgetragen und leicht einmassiert. Danach wurde die betroffene Stelle zusätzlich mit Sauerstoff (Sauerstoffgenerator, Fa. Meddrop Technology AG) im Laufe von 20 min begast. Die Behandlungen fanden normalerweise 2 bis 3 mal pro Woche statt. Die Erfolge wurden mit Hilfe der visuellen Analog-Skala (VAS) ermittelt und als Verringerung der Schmerzen erfasst.

**Tabelle 4: Indikationen, Fallzahl und Behandlungserfolge**

| Indikation | Patientenzahl | erfolgreich | erfolglos |
|---|---|---|---|
| Arthrose im oberen Sprunggelenk | 7 | 6 | 1 |
| Arthritis im Kniegelenk | 7 | 6 | 1 |
| Arthritis in der Hüfte | 7 | 6 | 1 |
| Insgesamt | 21 | 18 | 3 |

Die Tabelle zeigt, dass eine Erfolgsquote von 85,7% erreicht wurde.

### Beispiel 8. Therapie von erkrankten Pferden mit einem entzündlichen Hintergrund.

Alle Behandlungen wurden unter Anwendung einer COS-Boswellsäure haltigen Mikroemulsion durchgeführt. Die Mikroemulsion wurde in einer Menge von 0,5 bis 1,0 ml auf die betroffene Stelle mit dem Sprüher (Sauerstoffgenerator, Fa. Meddrop Technology AG) aufgetragen. Danach wurde die betroffene Stelle zusätzlich mit Sauerstoff (Sauerstoffgenerator, Fa. Meddrop Technology AG) im Laufe von 20 Minuten begast. Die Behandlungen fanden normalerweise 2 bis 3 mal pro Woche statt.

**Tabelle 5: Therapie von 15 erkrankten Pferden mit Entzündungen**

| Indikation | Krankheitsfälle | Behandlung erfolgreich | Behandlung erfolglos |
|---|---|---|---|
| Hufgelenksentzündungen | 7 | 6 | 1 |
| Fesselgelenksentzündungen | 8 | 7 | 1 |
| Gesamt | 15 | 13 (87 %) | 2(13%) |

Die Tabelle zeigt, dass eine Erfolgsquote von 87 % erreicht wurde.

### Beispiel 9

### Photodynamische Therapie (PDT) mit dem Addukt aus COS und 5-Aminolävulinsäure (ALA)

Der Nachweis der therapeutischen Wirkung von COS-ALA-Addukten erfolgte an insgesamt 60 Ratten mit einem Walker-Carciosarcom (30 Verum- und 30 Kontrolltiere). Die Tumore waren den Tieren implantiert worden, die photodynamische Therapie mit COS-ALA wurde durchgeführt, wenn die Tumoren eine Große von 1 cm³ erreicht hatten.

Zur Behandlung diente eine Lampe "AKTILITE" (Galdena AG, Frankfurt/M.) mit monochromatischem Licht der Wellenlänge λ = 634 nm. Die Bestrahlungsdosis betrug 37 J/cm², die mittlere Energie 8,5 W. Die behandelten Gruppen bestanden aus jeweils 6 Tieren, die nach 1, 2, 3, 5, 6 und 24 Stunden sowie 2 Wochen untersucht wurden. Dazu wurden die Tiere getötet, die Tumore exstirpiert und deren Gewicht und Größe gemessen. Für histologische Untersuchungen wurde Gewebe in 10 %iger Formalinlösung fixiert.

Das Gehalt an Porphyrin PP IX wurde mit Hilfe eines Spektrofluorimeters "LS 45 B" (Perkin Elmer) bestimmt (Anregung bei λ = 400 nm und Messung bei λ = 625 nm). Die Porphyrine wurden aus Tumorhomogenisaten mit Acetonitril/Ethanol(1+6)-Lösung extrahiert und die Konzentration aus Kalibrierkurven bestimmt.

Zur Bestimung des oxidativen Stress in den Tumorhomogenisaten diente der Gehalt an Malondialdehyd, der mit Hilfe der 2-Thiobarbitursäure bestimmt wurde.

Die Ergebnisse sind in Tabelle 6 dargestellt.

**Tabelle 6: Konzentration von PP IX und Malondialdehyd in Tumorhomogenisaten nach Behandlung der Tiere mit ALA und COS-ALA**

| Analyt | Behandlung | Zeit nach PDT | | | | | |
|---|---|---|---|---|---|---|---|
| | | 1 h | 2 h | 3 h | 5 h | 6 h | 24 h |
| PP IX | ALA | 3,9 | 2,8 | 1,7 | 0,4 | n.d. | n.d |
| | COS-ALA | 16,2 | 12,1 | 6,0 | 2,5 | 0,9 | n.d |
| Malondialdehyd, | ALA | 0,25 | 0,42 | 0,47 | 0,55 | 0,61 | 0,75 |
| nmol/mL | COS-ALA | 0,12 | 0,13 | 0,15 | 0,17 | 0,17 | 0,19 |

PP IX ist als dimensionslose Größe in Form des Verhältnisses der Konzentrationen im Tumorhomogenisat zum Plasma angegeben (n. d.: nicht detektierbar).

Die Tabelle 6 zeigt, dass COS-Präparate innerhalb von 6 Stunden zu statistisch signifikant höheren Porphyrinspiegeln in Tumorhomogenaten führen.

Die Kopplung von ALA an COS reduziert wesentlich die Bildung von Lipidperoxiden in den Tumoren (ermittelt durch die Bestimmung von Malondialdehyd) in allen Testintervallen.

Aus den Untersuchungsergebnissen ist auch eine synergistische Wirkung von COS-Präparaten zu sehen. Die Bestätigung des höheren Wirkpotentials der COS-ALA-Addukte zeigen die histologischen Untersuchungen. Die zytologischen Untersuchungen zeigen, dass in der Mitte des Tumorgewebes bei COS-ALA eine Nekrose deutlich nach 24 Stunden zu sehen ist, im Gegensatz zu ALA, wo diese Wirkung erst nach 72 Stunden zu sehen ist. Nach einer Woche verbreitet sich die Nekrose bei dem COS-ALA-Addukt auf das ganze Tumorgewebe, bei ALA erst nach 2 Wochen. Dies zeigt, dass COS-Präparate das Potential zur Verbesserung der Aufnahme in Tumorgewebe besitzen und trotz ihrer niedrigen antioxidativen Wirkung höhere tumornekrotische Wirkung zeigen.

## Patentansprüche

1. Zusammensetzung enthaltend ein Chitosan-Oligosaccharid mit einem mittleren Molekulargewicht von 240 bis 200.000 Dalton sowie mindestens ein Arzneimittel, wobei das Chitosan-Oligosaccharid und das Arzneimittel als Addukt vorliegen und die Adduktbildung auf Ionenbindung, Chelatbildung oder anderen Wechselwirkungen beruht, wobei es sich um eine Zusammensetzung zur transdermalen, topischen Anwendung am menschlichen oder tierischen Körper handelt, die Zusammensetzung als Mikroemulsion vorliegt und der Anteil des Arzneimittels in der Zusammensetzung 0,01 bis 40 Gew.-% bezogen auf das Chitosan-Oligosaccharid beträgt,
**dadurch gekennzeichnet, dass** die Zusammensetzung einen Acetatgehalt von 2 bis 5 Gew.-% bezogen auf das Chitosan-Oligosaccharid hat.

2. Zusammensetzung nach Anspruch 1, **dadurch gekennzeichnet, dass** das Arzneimittel ein entzündungshemmender Wirkstoff, ein Analgetikum, ein Cytostatikum, ein Photosensibilisator, ein Hautarzneimittel, ein Antiphlogistikum und/oder ein Antirheumatikum ist.

3. Zusammensetzung nach Anspruch 2, **dadurch gekennzeichnet, dass** das Antiphlogistikum oder das Antirheumatikum eine Verbindung aus der Gruppe der Glucocorticoide, Pyrazolidine, Essigsäurederivate, Oxicame, Coxibe, Salicylate, Immunsuppressiva/Immunomodulatoren, Fenamate oder Propionsäurederivate ist.

4. Zusammensetzung nach Anspruch 2, **dadurch gekennzeichnet, dass** das Analgetikum eine Verbindung aus der Gruppe der Opioide, Phenylpiperidinderivate, Diphenylpropylaminderivate, Benzomorphanderivate, Oripavinderivate oder Morphinanderivate ist.

5. Zusammensetzung nach einem der Ansprüche 2 bis 4, **dadurch gekennzeichnet, dass** das Analgetikum oder Antiphlogistikum ausgewählt ist aus der Gruppe:
Morphin, Codein, Dihydrocodein, Oxycodon, Fentanyl, Piritramid, Pethidin, Pentazocin, Methadon, Buprenorphin, Tramadol, Flupirtin, Nalbuphin, Nefopam, Metamizol, Paracetamol, Phenylbutazon, Mofebutazon, Acemetacin, Ibuprofen, Naproxen, Diclofenac, Ketoprofen, Ketorolac, Clofezol, Lonazolac, Aceclofenac, Indometacin, Sulindac, Tolmetin, Oxaprozin, Dexibuprofen, Mefenamin, Proglumetacin, Tiaprofensäure, Surprofen, Dexketoprofen, Lornoxicam, Meloxicam, Piroxicam, Penicillinamin, Chloroquin, Cromoglicinsäure, Nedocromil, Methotrexat, Auranofin, Natriumaurothiomalat, Oxaceprol, Leflunomid, Sulfasalazin, Valdecoxib, Celecoxib, Lumeracoxib, Ademetionin, Hyaluronsäure, Rofecoxib, Epibatidin, Boswellsäure, Methylsalicylsäure, Diflunisal, Phenazon, Proxyphenazon, Aescin, Escin, Heparin, Benzydamin, Bufexamac, Flurbiprofen, Oxyphenbutazon, Salicylamid, Tolfenaminsäure, Clobetasol, Triamcinolonacetonid, Triamcinolonhexacetonid, Betamethason, Dexamethason, Prednisolon, Hydrocortison, Fluticason, Liponsäure, Budesonid, Montelukast, Gentisinsäure, Salicylsäure, 5-Aminosalicylsäure, Olsalizin, Anthranilsäure, Flufenaminsäure sowie deren Säuren, Ester und Derivate.

6. Zusammensetzung nach Anspruch 1 oder 2, **dadurch gekennzeichnet dass** das Arzneimittel ein Phytopharmakum ist.

7. Zusammensetzung nach Anspruch 1, **dadurch gekennzeichnet, dass** das Arzneimittel ein Antifibrinolytikum, ein Hormon, eine Aminosäure, ein Peptid, ein Vitamin oder ein Antioxidans ist.

8. Zusammensetzung nach einem der Ansprüche 1 bis 7, **dadurch gekennzeichnet, dass** das Chitosan-Oligosaccharid durch Hydrolyse von Chitosan mittels eines Enzyms und Essigsäure erhalten wird.

9. Zusammensetzung nach einem der Ansprüche 1 bis 8, **dadurch gekennzeichnet, dass** der Anteil des Arzneimittels in der Zusammensetzung 0,05 bis 35 Gew.-%, insbesondere 0,1 bis 30 Gew.-% bezogen auf das Chitosan-Oligosaccharid beträgt.

10. Zusammensetzung nach einem der Ansprüche 1 bis 9, **dadurch gekennzeichnet, dass** das Chitosan-Oligosaccharid ein mittleres Molekulargewicht von 240 bis 50.000 Dalton aufweist.

11. Zusammensetzung nach einem der Ansprüche 1 bis 10 mit der Maßgabe, dass das Arzneimittel nicht aus der Gruppe Nicotinsäure, Acetylsalicylsäure, Milchsäure, Glutaminsäure, Nifluminsäure, Bernsteinsäure, Ascorbinsäure, Acexamsäure, Glycin, L-Carnitin, γ-Amino-β-phenylbernsteinsäure, Adenosinphosphat, Cocarboxylase, Aspartam, Gemfibrozil, Aminocapronsäure, Vitamin B₃, Methionin, einem Salz der vorgenannten Verbindungen, Antibiotika oder Polysaccharide ausgewählt ist.

## Claims

1. Composition comprising a chitosan oligosaccharide having a mean molecular weight of from 240 to 200,000 daltons and at least one medicament, wherein the chitosan oligosaccharide and the medicament are present in the form of an adduct and the adduct formation is based on ionic bonding, chelate formation or other interactions, wherein the composition is a composition for transdermal, topical use on the human or animal body, the composition is present in the form of a microemulsion, and the proportion of the medicament in the composition is from 0.01 to 40% by weight, based on the chitosan oligosaccharide,
**characterized in that**
the composition has an acetate content of from 2 to 5% by weight, based on the chitosan oligosaccharide.

2. Composition according to Claim 1, **characterized in that** the medicament is an anti-inflammatory active ingredient, an analgesic, a cytostatic, a photosensitizer, a dermatological medicament, an antiphlogistic and/or an antirheumatic.

3. Composition according to Claim 2, **characterized in that** the antiphlogistic or the antirheumatic is a compound from the group of the glucocorticoids, pyrazolidines, acetic acid derivatives, oxicams, coxibs, salicylates, immunosuppressants/- immunomodulators, fenamates or propionic acid derivatives.

4. Composition according to Claim 2, **characterized in that** the analgesic is a compound from the group of the opioids, phenylpiperidine derivatives, diphenylpropyl-amine derivatives, benzomorphan derivatives, oripavine derivatives or morphinan derivatives.

5. Composition according to one of Claims 2 to 4, **characterized in that** the analgesic or antiphlogistic is selected from the group:
morphine, codeine, dihydrocodeine, oxycodone, fentanyl, piritramide, pethidine, pentazocine, methadone, buprenorphine, tramadol, flupirtine, nalbuphine, nefopam, metamizole, paracetamol, phenylbutazone, mofebutazone, acemetacin, ibuprofen, naproxen, diclofenac, ketoprofen, ketorolac, clofezol, lonazolac, aceclofenac, indometacin, sulindac, tolmetin, oxaprozin, dexibuprofen, mefenamine, proglumetacin, tiaprofenic acid, surprofen, dexketoprofen, lornoxicam, meloxicam, piroxicam, penicillamine, chloroquine, cromoglicic acid, nedocromil, methotrexate, auranofin, sodium aurothiomalate, oxaceprol, leflunomide, sulfasalazine, valdecoxib, celecoxib, lumiracoxib, ademetionine, hyaluronic acid, rofecoxib, epibatadine, boswellic acid, methylsalicylic acid, diflunisal, phenazone, propyphenazone, aescin, escin, heparin, benzydamine, bufexamac, flurbiprofen, oxyphenbutazone, salicylamide, tolfenamic acid, clobetasol, triamcinolone acetonide, triamcinolone hexacetonide, betamethasone, dexamethasone, prednisolone, hydrocortisone, fluticasone, lipoic acid, budesonide, montelukast, gentisic acid, salicylic acid, 5-aminosalicylic acid, olsalazine, anthranilic acid, flufenamic acid, and acids, esters and derivatives thereof.

6. Composition according to Claim 1 or 2, **characterized in that** the medicament is a phyto-pharmaceutical.

7. Composition according to Claim 1, **characterized in that** the medicament is an antifibrinolytic, a hormone, an amino acid, a peptide, a vitamin or an antioxidant.

8. Composition according to one of Claims 1 to 7, **characterized in that** the chitosan oligosaccharide is obtained by hydrolysis of chitosan by means of an enzyme and acetic acid.

9. Composition according to one of Claims 1 to 8, **characterized in that** the proportion of the medicament in the composition is from 0.05 to 35% by weight, in particular from 0.1 to 30% by weight, based on the chitosan oligosaccharide.

10. Composition according to one of Claims 1 to 9, **characterized in that** the chitosan oligosaccharide has a mean molecular weight of from 240 to 50,000 daltons.

11. Composition according to one of Claims 1 to 10, with the proviso that the medicament is not selected from the group nicotinic acid, acetylsalicylic acid, lactic acid, glutamic acid, niflumic acid, succinic acid, ascorbic acid, acexamic acid, glycine, L-carnitine, γ-amino-β-phenylsuccinic acid, adenosine phosphate, cocarboxylase, aspartame, gemfibrozil, aminocaproic acid, vitamin B₃, methionine, a salt of the above-mentioned compounds, antibiotics or polysaccharides.

## Revendications

1. Composition contenant un oligosaccharide de chitosane présentant un poids moléculaire moyen de 240 à 200.000 daltons ainsi qu'au moins un médicament, l'oligosaccharide de chitosane et le médicament se trouvant sous forme de produit d'addition et la formation du produit d'addition reposant sur une liaison ionique, une formation de chélate ou d'autres interactions, la composition étant une composition pour l'utilisation transdermique, topique sur le corps humain ou animal, la composition se trouvant sous forme de microémulsion et la proportion de médicament dans la composition étant de 0,01 à 40% en poids par rapport à l'oligosaccharide de chitosane, **caractérisée en ce que** la composition présente une teneur en acétate de 2 à 5% en poids par rapport à l'oligosaccharide de chitosane.

2. Composition selon la revendication 1, **caractérisée en ce que** le médicament est une substance active anti-inflammatoire, un analgésique, un cytostatique, un agent de photosensibilisation, un médicament pour la peau, un antiphlogistique et/ou un antirhumatismal.

3. Composition selon la revendication 2, **caractérisée en ce que** l'antiphlogistique ou l'antirhumatismal est un composé du groupe formé par les glucocorticoïdes, les pyrazolidines, les dérivés de l'acide acétique, les oxicames, les coxibs, les salicylates, les immunosuppresseurs/immunomodulateurs, les fénamates ou les dérivés de l'acide propionique.

4. Composition selon la revendication 2, **caractérisée en ce que** l'analgésique est un composé du groupe formé par les opioïdes, les dérivés de phénylpipéridine, les dérivés de diphénylpropylamine, les dérivés de benzomorphane, les dérivés d'oripavine ou les dérivés de morphinane.

5. Composition selon l'une quelconque des revendications 2 à 4, **caractérisée en ce que** l'analgésique ou l'antiphlogistique est choisi dans le groupe formé par :
la morphine, la codéine, la dihydrocodéine, l'oxycodone, le fentanyl, le piritramide, la péthidine, la pentazocine, la méthadone, la buprénorphine, le tramadol, la flupirtine, la nalbuphine, le néfopam, le métamizol, le paracétamol, la phénylbutazone, la mofébutazone, l'acémétacine, l'ibuprofène, le naproxène, le diclofénac, le cétoprofène, le kétorolac, le clofézol, le lonazolac, l'acéclofénac, l'indométacine, le sulindac, la tolmétine, l'oxaprozine, le dexibuprofène, la méfénamine, la proglumétacine, l'acide tiaprofénique, le surprofène, le dexkétoprofène, le lornoxicam, le méloxicam, le piroxicam, la pénicillinamine, la chloroquine, l'acide cromoglicique, le nédocromil, le méthotrexate, l'auranofine, l'aurothiomalate de sodium, l'oxacéprol, le léflunomide, la sulfasalazine, le valdecoxib, le célécoxib, le lumiracoxib, l'adémétionine, l'acide hyaluronique, le rofécoxib, l'épibatidine, l'acide boswellique, l'acide méthylsalicylique, le diflunisal, la phénazone, la propyphénazone, l'aescine, l'escine, l'héparine, la benzydamine, le buféxamac, le flurbiprofène, l'oxyphénbutazone, le salicylamide, l'acide tolfénamique, le clobétasol, le triamcinolonacétonide, le triamcinolone hexacétonide, la bêtaméthasone, la dexaméthasone, la prednisolone, l'hydrocortisone, la fluticasone, l'acide liponique, le budésonide, la montelukast, l'acide gentisique, l'acide salicylique, l'acide 5-aminosalicylique, l'olsalazine, l'acide anthranilique, l'acide flufénamique ainsi que leurs acides, esters et dérivés.

6. Composition selon la revendication 1 ou 2, **caractérisée en ce que** le médicament est un agent phytopharmaceutique.

7. Composition selon la revendication 1, **caractérisée en ce que** le médicament est un antifibrinolytique, une hormone, un acide aminé, un peptide, une vitamine ou un antioxydant.

8. Composition selon l'une quelconque des revendications 1 à 7, **caractérisée en ce que** l'oligosaccharide de chitosane est obtenu par hydrolyse de chitosane au moyen d'une enzyme et d'acide acétique.

9. Composition selon l'une quelconque des revendications 1 à 8, **caractérisée en ce que** la proportion de médicament dans la composition est de 0,05 à 35% en poids, en particulier de 0,1 à 30% en poids, par rapport à l'oligosaccharide de chitosane.

10. Composition selon l'une quelconque des revendications 1 à 9, **caractérisée en ce que** l'oligosaccharide de chitosane présente un poids moléculaire moyen de 240 à 50.000 daltons.

11. Composition selon l'une quelconque des revendications 1 à 10, à condition que le médicament ne soit pas choisi dans le groupe formé par l'acide nicotinique, l'acide acétylsalicylique, l'acide lactique, l'acide glutamique, l'acide niflumique, l'acide succinique, l'acide ascorbique, l'acide acéxamique, la glycine, la L-carnitine, l'acide γ-amino-ß-phénylsuccinique, l'adénosine phosphate, la cocarboxylase, l'aspartame, le gemfibrozil, l'acide aminocaproïque, la vitamine B₃, la méthionine, un sel des composés susmentionnés, les antibiotiques ou les polysaccharides.
